# EUROPEAN PATENT SPECIFICATION

(11) **EP 3 388 027 B1**
(45) Date of publication and mention of the grant of the patent: **30.11.2022**
(21) Application number: 18160595.7
(22) Date of filing: 30.05.2014
(51) Int. Cl.: A61F 2/24

(54) **STRUCTURAL MEMBERS FOR PROSTHETIC MITRAL VALVES**
BAUTEILE FÜR MITRALKLAPPENPROTHESEN
ELÉMENTS STRUCTURAUX POUR PROTHÈSES DE VALVULE MITRALE

(30) Priority: 30.05.2013 US 201361829076 P; 15.01.2014 US 201414155417
(43) Date of publication of application: 17.10.2018
(62) Divisional of application: 14734333.9
(73) Proprietor: Tendyne Holdings, Inc., Roseville, MN 55113 (US)
(72) Inventor: CHRISTIANSON, Mark, Plymouth, MN 55441 (US); PERRIN, Chad, Andover, MN 55304 (US); TEGELS, Zachary, Minneapolis, MN 55419 (US); EKVALL, Craig, East Bethel, MN 55092 (US); VIDLUND, Robert, Forest Lake, MN 55025 (US)
(74) Representative: Gill Jennings & Every LLP

(56) References cited:
- WO-A2-2012/177942
- WO-A2-2013/028387
- US-A1- 2012 215 303

## Description

### Background

### Field of Invention

An improved transcatheter prosthetic heart valve includes structural members, such as in the form of wire frames, which provide support for the valve and aid in reducing or preventing leakage.

### Background

Valvular heart disease and specifically aortic and mitral valve disease is a significant health issue in the US. Annually approximately 90,000 valve replacements are conducted in the US. Traditional valve replacement surgery, the orthotopic replacement of a heart valve, is an "open heart" surgical procedure. Briefly, the procedure necessitates a surgical opening of the thorax, initiation of extra-corporeal circulation with a heart-lung machine, stopping and opening the heart, excision and replacement of the diseased valve, and re-starting of the heart. While valve replacement surgery typically carries a 1-4% mortality risk in otherwise healthy persons, a significantly higher morbidity is associated to the procedure, largely due to the necessity for extra-corporeal circulation. Further, open heart surgery is often poorly tolerated in elderly patients.

Thus if the extra-corporeal component of the procedure could be eliminated, morbidities and cost of valve replacement therapies would be significantly reduced.

While replacement of the aortic valve in a transcatheter manner is the subject of intense investigation, lesser attention has been focused on the mitral valve. This is in part reflective of the greater level of complexity associated to the native mitral valve apparatus and thus a greater level of difficulty with regards to inserting and anchoring the replacement prosthesis.

Various problems exist in this field, including problems of insufficient articulation and sealing of the valve within the native annulus, pulmonary edema due to poor atrial drainage, perivalvular leaking around the installed prosthetic valve, lack of a good fit for the prosthetic valve within the native mitral annulus, atrial tissue erosion, excess wear on the valve structures, interference with the aorta at the posterior side of the mitral annulus, and lack of customization, to name a few. Accordingly, there is still a need for an improved prosthetic mitral valve.

US 2012/215303 discloses a replacement heart valve and method of implanting the replacement heart valve.

### Summary

The invention is defined by claim 1 and it consists of an apparatus comprising an outer frame and an inner frame, the inner frame supporting a prosthetic heart valve leaflet assembly. The prosthetic cardiovascular valve includes a cylindrical framework defining a lumen. The cylindrical framework includes multiple generally diamond-shaped members. Each diamond-shaped member defines multiple lateral vertices and multiple longitudinal vertices. Each diamond-shaped member is coupled to one or more other diamond-shaped members. Each coupling can be at or about each of the lateral vertices of the diamond-shaped member.

### Brief Description of the Drawings

FIG. 1 is an exploded view of a prosthetic cardiovascular valve according to an embodiment.
FIG. 2 is an oblique projection of a three-diamond self-expanding wire frame as a cylindrical frame defining a lumen according to an embodiment.
FIG. 3 is a perspective side view of a three-diamond self-expanding wire frame as a cylindrical frame defining a lumen according to another embodiment.
FIG. 15 is an exploded view of a prosthetic cardiovascular valve according to another embodiment.
FIG. 16 is an opened and flattened view of an unexpanded inner wireframe structure, according to an embodiment.
FIGs. 17 and 18 are side and bottom views, respectively, of the inner wireframe structure of FIG. 16 in an expanded configuration.
FIG. 19 is an opened and flattened view of an unexpanded outer frame according to an embodiment.
FIGs. 20 and 21 are side and top views, respectively, of the outer frame of FIG. 19 in an expanded configuration.
FIGs. 22-24 are side, front, and top views of an assembly of the inner wireframe structure of FIGs. 16-18 and the outer frame of FIGs. 19-21, forming a support structure for a prosthetic valve, according to an embodiment.

### Detailed Description

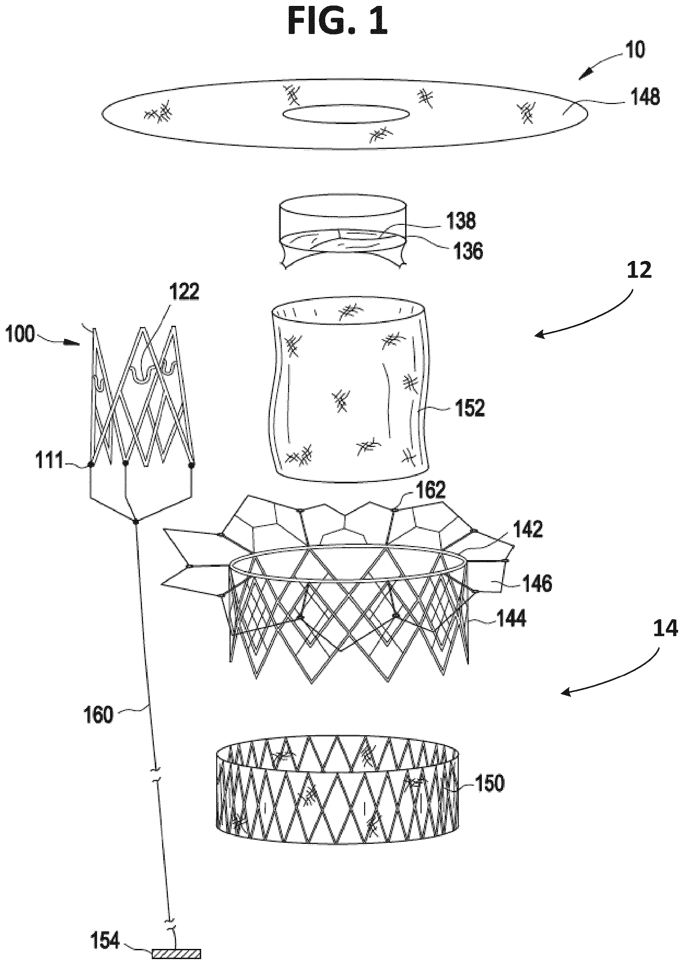

FIG. 1 is an exploded view of one embodiment of a pre-configured compressible transcatheter prosthetic cardiovascular valve 10. In this embodiment, valve 10 includes an inner structure or assembly 12, and an outer structure or assembly 14. Valve 10 may be coupled to a tether 160, and a tether anchor 154.

Inner assembly 12 includes a self-expanding inner frame 100, an outer cylindrical wrap 152 disposed about the inner wire frame (to acts as a cover to prevent valvular leakage) and a leaflet structure 136 (comprised of articulating leaflets 138 that define a valve function). The leaflet structure 136 may be sewn to the inner frame 100. The inner frame 100 also has (tether) attachment apertures 111 to which the tether 160 can be attached. Tether 160 is connected to tether anchor 154, which in this embodiment is implemented as an epicardial securing pad.

Outer assembly 14 includes an outer stent or frame 144, an outer cover 150, and a cuff covering 148. In this embodiment, outer frame 144 has a flared, articulating collar or

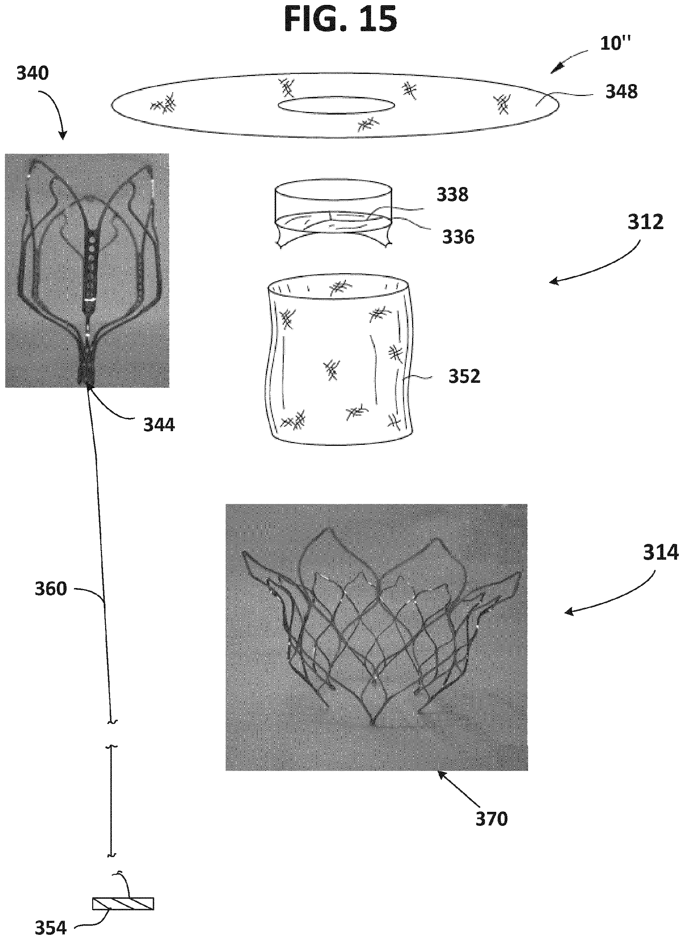

FIG. 15 is an exploded view of a prosthetic cardiovascular valve according to another embodiment.

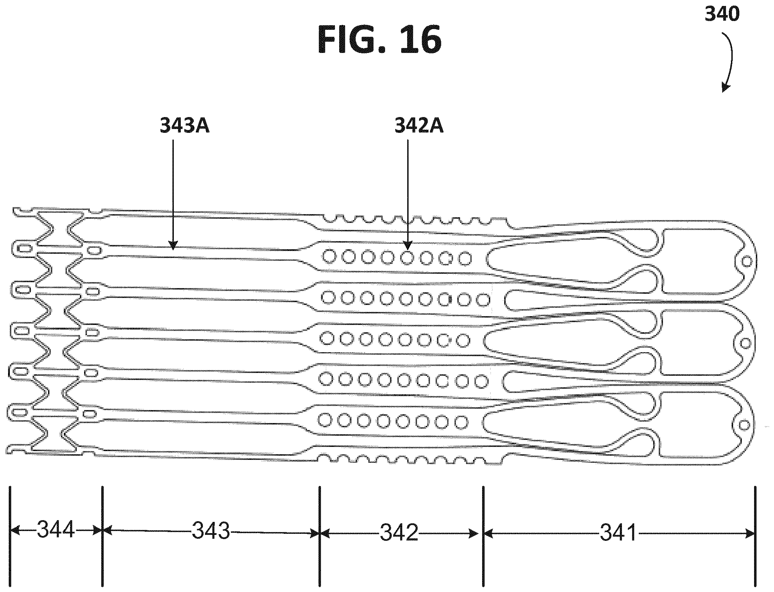

FIG. 16 is an opened and flattened view of an unexpanded inner wireframe structure, according to an embodiment.

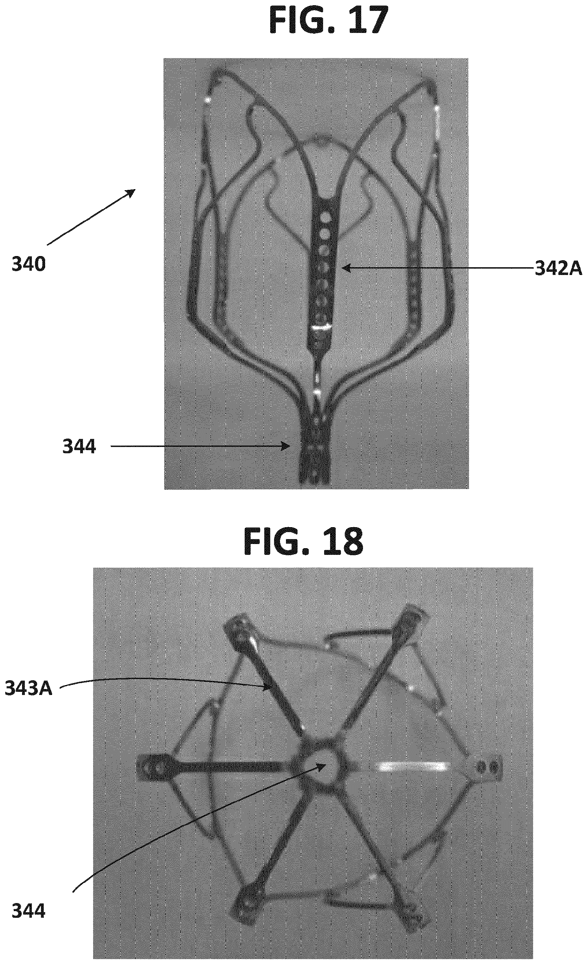

FIGs. 17 and 18 are side and bottom views, respectively, of the inner wireframe structure of FIG. 16 in an expanded configuration.

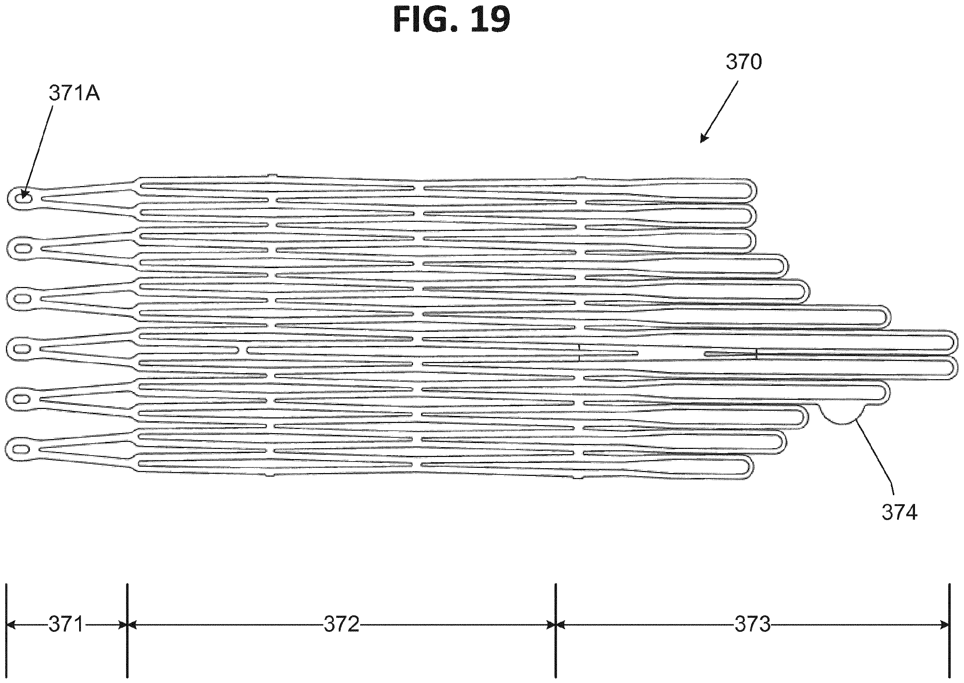

FIG. 19 is an opened and flattened view of an unexpanded outer frame according to an embodiment.

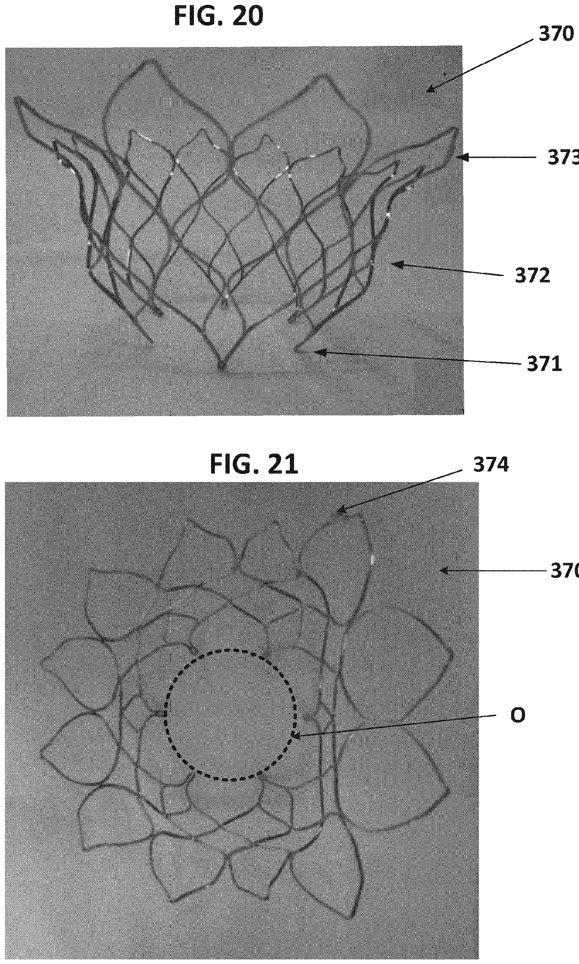

FIGs. 20 and 21 are side and top views, respectively, of the outer frame of FIG. 19 in an expanded configuration.

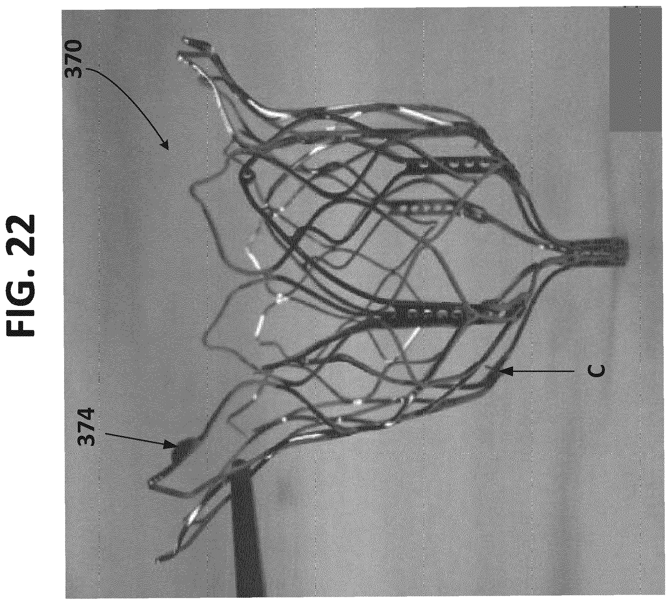

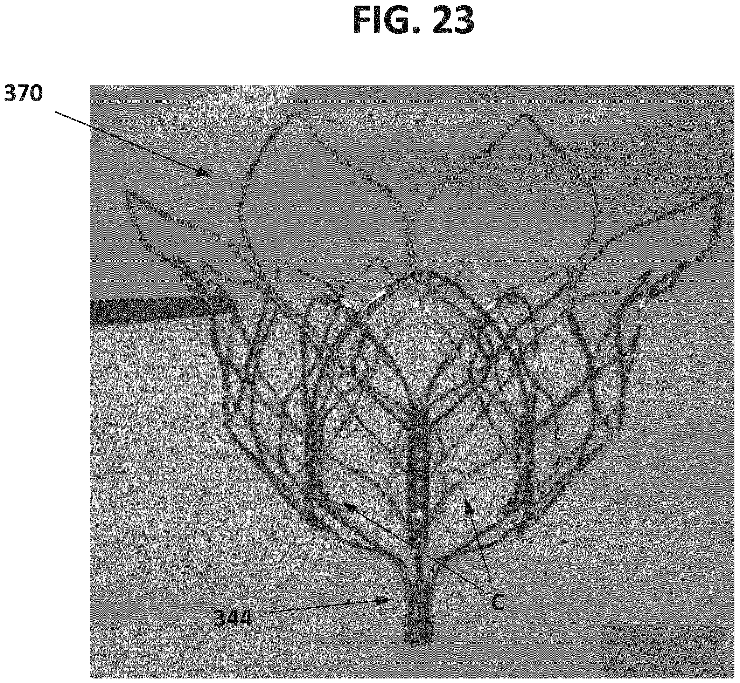

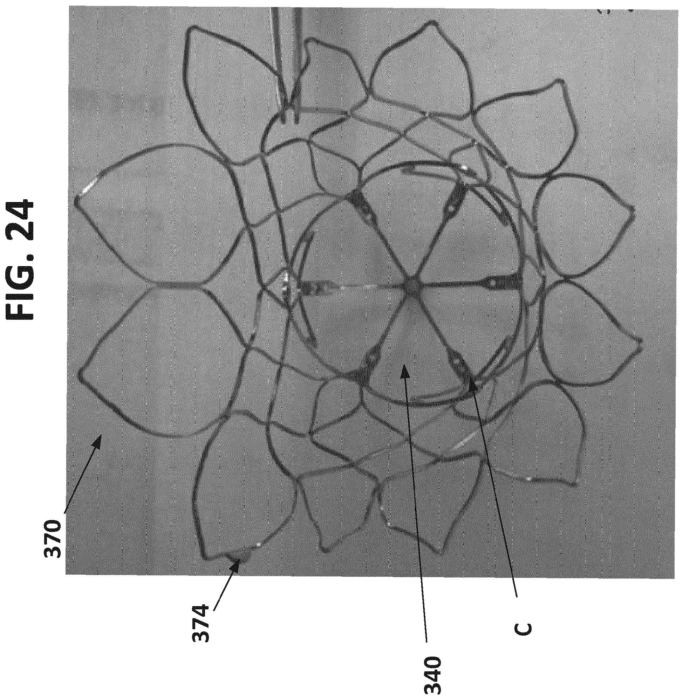

FIGs. 22-24 are side, front, and top views of an assembly of the inner wireframe structure of FIGs. 16-18 and the outer frame of FIGs. 19-21, forming a support structure for a prosthetic valve, according to an embodiment.

### Detailed Description

FIG. 1 is an exploded view of one embodiment of a pre-configured compressible transcatheter prosthetic cardiovascular valve 10. In this embodiment, valve 10 includes an inner structure or assembly 12, and an outer structure or assembly 14. Valve 10 may be coupled to a tether 160, and a tether anchor 154.

Inner assembly 12 includes a self-expanding frame 100, an outer cylindrical wrap 152 disposed about the inner wire frame (to acts as a cover to prevent valvular leakage) and a leaflet structure 136 (comprised of articulating leaflets 138 that define a valve function). The leaflet structure 136 may be sewn to the inner wireframe 100. The wireframe 100 also has (tether) attachment apertures 111 to which the tether 160 can be attached. Tether 160 is connected to tether anchor 154, which in this embodiment is implemented as an epicardial securing pad.

Outer assembly 14 includes an outer stent or frame 144, an outer cover 150, and a cuff covering 148. In this embodiment, outer frame 144 has a flared, articulating collar or cuff 146 over which the cuff covering 148 is disposed. Cuff 146 has a D-shaped section 162 to accommodate and solve left ventricular outflow tract (LVOT) obstruction issues.

Cuff 146 may be configured a substantially flat plate that projects beyond the diameter of the tubular body of outer frame 144 to form a rim or border. The terms flared end, cuff, flange, collar, bonnet, apron, or skirting used interchangeable herein. When the tubular body of frame 144 is pulled through the aperture of a mitral valve aperture, the mitral annulus, such as by tether loops, in the direction of the left ventricle, the cuff acts as a collar to stop the frame from traveling any further through the mitral valve aperture. The entire prosthetic valve is held by longitudinal forces between the cuff, which is seated in the left atrium and mitral annulus, and the ventricular tethers attached to the left ventricle.

Cuff 146 may be formed from a stiff, flexible shape-memory material such as the nickel-titanium alloy material Nitinol^{®} formed as wire, and covered by cuff covering 148, which may be formed of stabilized tissue or other suitable biocompatible or synthetic material. In one embodiment, the cuff is constructed from independent articulating radial tines or posts of wire extending axially around the circumference of the bend or seam where cuff 146 transitions to the tubular body of frame 144 (in an integral flared end or cuff) or where cuff 146 is attached to the frame body (in an implementation in which they are separate, but joined components).

With cuff cover 148 in place articulating radial tines or posts of wire provide the cuff the ability to move up and down, to articulate, along the longitudinal axis that runs through the center of frame 144. In other words, the individual articulating radial tines or posts of wire can independently move up and down, and can spring back to their original position due to the relative stiffness of the wire. The tissue or material that covers the cuff wire has a certain modulus of elasticity such that, when attached to the wire of the cuff, is able to allow the wire spindles to move. This flexibility gives the cuff, upon being deployed within a patient's heart, the ability to conform to the anatomical shape necessary for a particular application. In the example of a prosthetic mitral valve, the cuff is able to conform to the irregularities of the left atrium and shape of the mitral annulus, and to provide a tight seal against the atrial tissue adjacent the mitral annulus and the tissue within the mitral annulus. As stated previously, this feature provides a degree of flexibility in sizing the mitral valve and prevents blood from leaking around the implanted prosthetic heart valve.

An additional aspect of the cuff dimension and shape is that, when fully seated and secured, the edge of the cuff preferably should not be oriented laterally into the atrial wall, in which orientation it might produce a penetrating or cutting action on the atrial wall.

In some embodiments, the wire spindles of the cuff are substantially uniform in shape and size. In some embodiments, each loop or spindle may be of varying shapes and sizes. In this example, it is contemplated that the articulating radial tines or posts of wire may form a pattern of alternating large and small articulating radial tines or posts of wire, depending on where the valve is being deployed. In the case of a prosthetic mitral valve, preoperative imaging may allow for customizing the structure of the cuff depending on a particular patient's anatomical geometry in the vicinity of the mitral annulus.

The cuff is constructed so as to provide sufficient structural integrity to withstand the intracardiac forces without collapsing.

Inner frame 100 and outer frame or frame 144, including cuff 146, are preferably formed to be deformed (compressed and/or expanded) and, when released, return to their original (undeformed) shapes. To achieve this, the components are preferably formed of materials, such as metals or plastics, that have shape memory properties. With regards to metals, Nitinol^{®} has been found to be especially useful since it can be processed to be austenitic, martensitic or super elastic. Martensitic and super elastic alloys can be processed to demonstrate the required compression features. Thus, inner frame 100 and outer frame or frame 144, including cuff 146, are preferably constructed of Nitinol^{®}, and are capable of maintaining their functions while under longitudinal forces that might cause a structural deformation or valve displacement. Other shape memory alloys, such as Cu-Zn-Al-Ni alloys, and Cu-Al-Ni alloys, may be used.

Inner frame 100 and outer frame or frame 144, including cuff 146, are preferably formed from a laser cut, thin-walled tube of Nitinol^{®}. The laser cuts form regular cutouts in the thin Nitinol^{®} tube. Secondarily the tube is placed on a mold of the desired shape, heated to the martensitic temperature and quenched. The treatment of the frame in this manner will form a flared end or cuff that has shape memory properties and will readily revert to the memory shape at the calibrated temperature.

Alternatively, these components may be constructed from braided wire

The cuff provides several functions. The first function is to inhibit perivalvular leakage and regurgitation of blood around the prosthesis. By flexing and sealing across the irregular contours of the annulus and atrium, leakage is minimized or prevented.

The second function of the cuff is to provide an adjustable and/or compliant bioprosthetic valve. The heart and its structures undergo complex conformational changes during the cardiac cycle. For example, the mitral valve annulus has a complex geometric shape known as a hyperbolic paraboloid that is shaped like a saddle, with the horn being anterior, the seat back being posterior, and the left and right valleys located medially and laterally. Beyond this complexity, the area of the mitral annulus changes over the course of the cardiac cycle. Further, the geometry of the tricuspid valve and tricuspid annulus continues to be a topic of research, posing its own particular problems. Accordingly, compliance is a very important but unfortunately often overlooked requirement of cardiac devices. Compliance here refers to the ability of the valve to change conformation with the native annulus in order to maintain structural position and integrity throughout the cardiac cycle. Compliance with the motion of the heart is a particularly useful feature, especially the ability to provide localized compliance where the underlying surfaces are acting differently from the adjacent surfaces. This ability to vary throughout the cardiac cycle allows the valve to remain seated and properly deployed in a manner not heretofore provided.

Additionally, compliance may be achieved through the use of the tethers where the tethers are preferably made from an elastic material. Tether-based compliance may be used alone, or in combination with the cuff-based compliance.

The third function of the cuff is to enable the valve, during implantation surgery, to conform to the irregular surfaces of the atrium. This function can be enhanced by the use of independent tethers, allowing for side-to-side fitting of the valve within the annulus. For example, where three tethers are used, they can be spaced circumferentially about 120 degrees relative to each other, which allows the surgeon to observe whether or where perivalvular leaking might be occurring and to pull on one side or the other to create localized pressure and reduce or eliminate the leakage.

The fourth function of the cuff is to counter the forces that act to displace the prosthesis toward/into the ventricle (i.e. atrial pressure and flow-generated shear stress) during ventricular filling.

The heart is known to generate an average left atrial pressure between about 8 and 30 mm Hg (about 0.15 to 0.6 psi). This left atrial filling pressure is the expected approximate pressure that would be exerted in the direction of the left ventricle when the prosthesis is open against the outer face of the flared end or cuff as an anchoring force holding the flared end or cuff against the atrial tissue that is adjacent the mitral valve. Cuff 146 counteracts this longitudinal pressure against the prosthesis in the direction of the left ventricle to keep the valve from being displaced or slipping into the ventricle. In contrast, left ventricular systolic pressure, normally about 120 mm Hg, exerts a force on the closed prosthesis in the direction of the left atrium. The tethers counteract this force and are used to maintain the valve position and withstand the ventricular force during ventricular contraction or systole. Accordingly, cuff 146 has sufficient structural integrity to provide the necessary tension against the tethers without being dislodged and pulled into the left ventricle. After a period of time, changes in the geometry of the heart and/or fibrous adhesion between prosthesis and surrounding cardiac tissues may assist or replace the function of the ventricular tethers in resisting longitudinal forces on the valve prosthesis during ventricular contraction.

Additional features of the cuff include that it functions to strengthen the leaflet assembly/frame complex by providing additional structure. Further, during deployment, the cuff functions to guide the entire structure, the prosthetic valve, into place at the mitral annulus during deployment and to keep the valve in place once it is deployed. Another important function is to reduce pulmonary edema by improving atrial drainage.

The valve leaflets are held by, or within, a leaflet assembly. In some embodiments, the leaflet assembly comprises a leaflet wire support structure to which the leaflets are attached and the entire leaflet assembly is housed within the frame body. In this embodiment, the assembly is constructed of wire and stabilized tissue to form a suitable platform for attaching the leaflets. In this aspect, the wire and stabilized tissue allow for the leaflet structure to be compressed when the prosthetic valve is compressed within the deployment catheter, and to spring open into the proper functional shape when the prosthetic valve is opened during deployment. In this embodiment, the leaflet assembly may optionally be attached to and housed within a separate cylindrical liner made of stabilized tissue or material, and the liner is then attached to line the interior of the frame body.

In this embodiment, the leaflet wire support structure is constructed to have a collapsible/expandable geometry. In some embodiments, the structure is a single piece of wire. The wireform is, in one embodiment, constructed from a shape memory alloy such as Nitinol^{®}. The structure may optionally be made of a plurality of wires, including between 2 to 10 wires. Further, the geometry of the wire form is without limitation, and may optionally be a series of parabolic inverted collapsible arches to mimic the saddle-like shape of the native annulus when the leaflets are attached. Alternatively, it may optionally be constructed as collapsible concentric rings, or other similar geometric forms, each of which is able to collapse or compress, then expand back to its functional shape. In some embodiments, there may be 2, 3 or 4 arches. In another embodiment, closed circular or ellipsoid structure designs are contemplated. In another embodiment, the wire form may be an umbrella-type structure, or other similar unfold-and-lock-open designs. In some embodiments utilizes super elastic Nitinol^{®} wire approximately 0.381mm (0.015") in diameter. In this embodiment, the wire is wound around a shaping fixture in such a manner that 2-3 commissural posts are formed. The fixture containing the wrapped wire is placed in a muffle furnace at a pre-determined temperature to set the shape of the wire form and to impart its super elastic properties. Secondarily, the loose ends of the wireform are joined with a stainless steel or Nitinol^{®} tube and crimped to form a continuous shape. In some embodiments, the commissural posts of the wireform are adjoined at their tips by a circular connecting ring, or halo, whose purpose is to minimize inward deflection of the post(s).

In some embodiments, the leaflet assembly is constructed solely of stabilized tissue or other suitable material without a separate wire support structure. The leaflet assembly in this embodiment is also disposed within the lumen of the frame and is attached to the frame to provide a sealed joint between the leaflet assembly and the inner wall of the frame. By definition, it is contemplated within the scope of the invention that any structure made from stabilized tissue and/or wire(s) related to supporting the leaflets within the frame constitute a leaflet assembly. In this embodiment, stabilized tissue or suitable material may also optionally be used as a liner for the inner wall of the frame and is considered part of the leaflet assembly.

Liner tissue or biocompatible material may be processed to have the same or different mechanical qualities, such as thickness, durability, etc., from the leaflet tissue.

Multiple types of tissue and biocompatible material may be used to cover the cuff, to form the valve leaflets, to form a wireless leaflet assembly, and/or to line both the inner and/or outer lateral walls of outer frame 144. As stated previously, the leaflet component may be constructed solely from stabilized tissue, without using wire, to create a leaflet assembly and valve leaflets. In this aspect, the tissue-only leaflet component may be attached to the frame with or without the use of the wire form. In some embodiments, there can be anywhere from 1, 2, 3 or 4 leaflets, or valve cusps.

The tissue may be used to cover the inside of the frame body, the outside of the frame body, and the top and/or bottom side of the cuff wire form, or any combination thereof.

In some embodiments, the tissue used herein is optionally a biological tissue and may be a chemically stabilized valve of an animal, such as a pig. In some embodiments, the biological tissue is used to make leaflets that are sewn or attached to a metal frame. This tissue is chemically stabilized pericardial tissue of an animal, such as a cow (bovine pericardium) or sheep (ovine pericardium) or pig (porcine pericardium) or horse (equine pericardium).

Preferably, the tissue is bovine pericardial tissue. Examples of suitable tissue include that used in the products Duraguard^{®}, Peri-Guard^{®}, and Vascu-Guard^{®}, all products currently used in surgical procedures, and which are marketed as being harvested generally from cattle less than 30 months old.

In some embodiments, the valve leaflets may optionally be made from a synthetic material such as polyurethane or polytetrafluoroethylene. Where a thin, durable synthetic material is contemplated, e.g. for covering the flared end or cuff, synthetic polymer materials such expanded polytetrafluoroethylene or polyester may optionally be used. Other suitable materials may optionally include thermoplastic polycarbonate urethane, polyether urethane, segmented polyether urethane, silicone polyether urethane, silicone-polycarbonate urethane, and ultra-high molecular weight polyethylene. Additional biocompatible polymers may optionally include polyolefins, elastomers, polyethylene-glycols, polyethersulphones, polysulphones, polyvinylpyrrolidones, polyvinylchlorides, other fluoropolymers, silicone polyesters, siloxane polymers and/or oligomers, and/or polylactones, and block co-polymers using the same.

In another embodiment, the valve leaflets may optionally have a surface that has been treated with (or reacted with) an anti-coagulant, such as, without limitation, immobilized heparin. Such currently available heparinized polymers are known and available to a person of ordinary skill in the art.

Alternatively, the valve leaflets may optionally be made from pericardial tissue or small intestine submucosal tissue.

In another embodiment, the prosthetic valve is sized and configured for use in areas other than the mitral annulus, including, without limitation, the tricuspid valve between the right atrium and right ventricle. Alternative embodiments may optionally include variations to the flared end or cuff structure to accommodate deployment to the pulmonary valve between the right ventricle and pulmonary artery, and the aortic valve between the left ventricle and the aorta. In one embodiment, the prosthetic valve is optionally used as a venous backflow valve for the venous system, including without limitation the vena cava, femoral, subclavian, pulmonary, hepatic, renal and cardiac. In this aspect, the flared end or cuff feature is utilized to provide additional protection against leaking.

As shown in FIG. 1, tether 160 may be attached to valve 10. Tether 160 (which may include multiple tethers) may extend to one or more tissue anchor locations within the heart. In some embodiments, the tether(s) extends downward through the left ventricle, exiting the left ventricle at the apex of the heart to be fastened on the epicardial surface outside of the heart. Similar anchoring is contemplated herein as it regards the tricuspid, or other valve structure requiring a prosthetic. There may be from 1 to 8, or more, tethers.

In some embodiments, the tether(s) may optionally be attached to the cuff to provide additional control over position, adjustment, and compliance. In some embodiments, one or more tethers are optionally attached to the cuff, in addition to, or optionally, in place of, the tethers attached to the outer frame 144. By attaching to the cuff and/or the frame, an even higher degree of control over positioning, adjustment, and compliance is provided to the operator during deployment.

During deployment, the operator is able to adjust or customize the tethers to the correct length for a particular patient's anatomy. The tether(s) also allows the operator to tighten the cuff onto the tissue around the valvular annulus by pulling the tether(s), which creates a leak-free seal.

In some embodiments, the tether(s) is optionally anchored to other tissue location(s) depending on the particular application of valve 10. In the case of a mitral valve, or the tricuspid valve, one or more tethers are optionally anchored to one or both papillary muscles, septum, and/or ventricular wall.

In some embodiments, the ventricular end of outer frame 144, or of inner frame 100, comes to 2-5 points onto which anchoring sutures or tether are affixed. The tethers will traverse the ventricle and ultimately be anchored to the epicardial surface of the heart approximately at the level of the apex. The tethers when installed under slight tension will serve to hold the valve in place, i.e. inhibit perivalvular leakage during systole.

The tethers, in conjunction with the cuff, provide greater compliance for the valve. The tethers may be made from surgical-grade materials such as biocompatible polymer suture material. Non-limiting examples of such material include ultra high-molecular weight polyethylene (UHMWPE), 2-0 exPFTE (polytetrafluoroethylene) or 2-0 polypropylene. In one embodiment the tethers are inelastic. One or more of the tethers may optionally be elastic to provide an even further degree of compliance of the valve during the cardiac cycle. Upon being drawn to and through the apex of the heart, the tethers may be fastened by a suitable mechanism such as tying off to a pledget or similar adjustable button-type anchoring device to inhibit retraction of the tether back into the ventricle. It is also contemplated that the tethers might be bioresorbable/bioabsorbable and thereby provide temporary fixation until other types of fixation take hold such a biological fibrous adhesion between the tissues and prosthesis and/or radial compression from a reduction in the degree of heart chamber dilation.

Valve 10 may optionally be deployed with a combination of installation tethers and permanent tethers, attached to outer frame 144, and/or cuff 146, and/or inner frame 100, the installation tethers being removed after the valve is successfully deployed. It is also contemplated that combinations of inelastic and elastic tethers may optionally be used for deployment and to provide structural and positional compliance of the valve during the cardiac cycle.

Valve 10 may be deployed as a prosthetic mitral valve using catheter delivery techniques. The entire valve 10 is compressed within a narrow catheter and delivered to the annular region of the native valve, preferably the left atrium, with a pre-attached tether apparatus. There, the valve 10 is pushed out of the catheter where it springs open into its preformed functional shape without the need for manual expansion using an inner balloon catheter. When the valve 10 is pulled into place, the outer frame 144 is seated in the native mitral annulus, leaving the cuff 146 to engage the atrial floor and prevent pull-through (where the valve is pulled into the ventricle). The native leaflets are not cut-away as has been taught in prior prosthetic efforts, but are used to provide a tensioning and sealing function around the outer frame 144. The valve 10 is preferably deployed asymmetrically to address LVOT problems, unlike non-accommodating prosthetic valves that push against the A2 anterior segment of the mitral valve and close blood flow through the aorta, which anatomically sits immediately behind the A2 segment of the mitral annulus. Thus, D-shaped section 162 is preferably deployed immediately adjacent/contacting the A2 segment since the flattened D-shaped section 162 is structurally smaller and has a more vertical profile (closer to paralleling the longitudinal axis of the outer frame) and thereby exerts less pressure on the A2 segment. Once valve 10 is properly seated, tether 160 may be extended out through the apical region of the left ventricle and secured using an epicardial pad 154 or similar suture-locking attachment mechanism.

Valve 10 is, in one embodiment, apically delivered through the apex of the left ventricle of the heart using a catheter system. In one aspect of the apical delivery, the catheter system accesses the heart and pericardial space by intercostal delivery. In another delivery approach, the catheter system delivers valve 10 using either an antegrade or retrograde delivery approach using a flexible catheter system, and without requiring the rigid tube system commonly used. In another embodiment, the catheter system accesses the heart via a trans-septal approach.

In some embodiments, the frame body extends into the ventricle about to the edge of the open mitral valve leaflets (approximately 25% of the distance between the annulus and the ventricular apex). The open native leaflets lay against the outside frame wall and parallel to the long axis of the frame (i.e. the frame holds the native mitral valve open).

In some embodiments, the diameter should approximately match the diameter of the mitral annulus. Optionally, the valve may be positioned to sit in the mitral annulus at a slight angle directed away from the aortic valve such that it is not obstructing flow through the aortic valve. Optionally, the outflow portion (bottom) of the frame should not be too close to the lateral wall of the ventricle or papillary muscle as this position may interfere with flow through the prosthesis. As these options relate to the tricuspid, the position of the tricuspid valve may be very similar to that of the mitral valve.

In one embodiment, to control the potential tearing of tissue at the apical entry point of the delivery system, a circular, semi-circular, or multi-part pledget may be employed. The pledget may be constructed from a semi-rigid material such as PTFE felt. Prior to puncturing of the apex by the delivery system, the felt is firmly attached to the heart such that the apex is centrally located. Secondarily, the delivery system is introduced through the central area, or orifice as it may be, of the pledget. Positioned and attached in this manner, the pledget acts to control any potential tearing at the apex.

In another embodiment the valve can be seated within the valvular annulus through the use of tines or barbs. These may be used in conjunction with, or in place of one or more tethers. The tines or barbs are located to provide attachment to adjacent tissue. In some embodiments, the tines are optionally circumferentially located around the bend/transition area between frame body 144 and the cuff 146. Such tines are forced into the annular tissue by mechanical means such as using a balloon catheter. In one non-limiting embodiment, the tines may optionally be semi-circular hooks that upon expansion of the frame body, pierce, rotate into, and hold annular tissue securely.

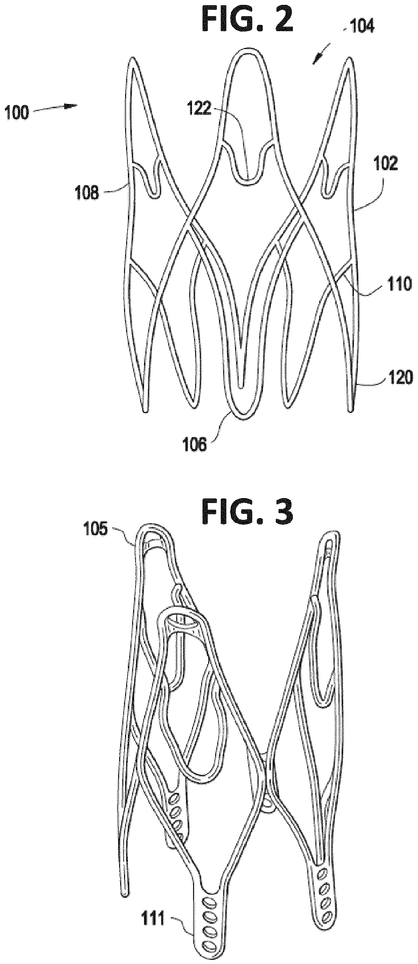

One embodiment of an inner frame 100 is shown in side view in FIG. 2. Frame 100 includes a cylindrical framework 102 defining a lumen 104, and including three generally diamond-shaped members 106, 108, 110. Each diamond-shaped member is directly connected to, or has at least one connecting member 120 connecting to, each of the other two diamond-shaped members. Spanning members 122 cross the open span of the diamond-shaped members and provide a strengthening structural enhancement, another sewing anchor location for the other components of inner assembly 12, or both.

Another embodiment of inner frame 100 is shown in side view in FIG. 3. This embodiment includes optional valve sewing rings 105 and tether attachment structures 111. Each valve sewing ring 105 provides an aperture for sewing the leaflet tissue structures to the wire framework 100.

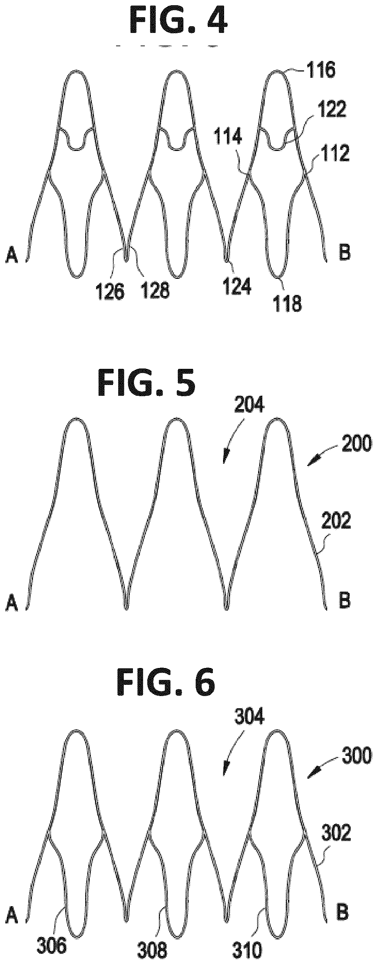

Another embodiment of inner frame 100 is shown in an opened and flattened view in FIG. 4. This view is intended primarily for illustrative purposes of the wireframe structure, since the manufacture of the cylindrical framework will generally be made from a laser-cut piece of Nitinol^{®} tubing that is expanded to form a larger cylindrical structure, and the wireframe structure will generally not be manufactured from a rolled-up welded metal lattice. FIG. 4 shows each diamond-shaped member defining two lateral vertices 112 and 114 and two longitudinal vertices 116 and 118. Each diamond-shaped member is directly connected to, or has at least one connecting member 120 connecting to, each of the other two diamond-shaped members. The connecting members defined in this embodiment as joined legs 126, 128 connected at a V-shaped connecting vertex 124. FIG. 4 also shows spanning members 122 crossing the open span of the diamond-shaped members and providing a strengthening structural enhancement, another sewing anchor location, or both. FIG. 4 shows point A and point B, which are the locations at which the connecting members are joined to form a cylindrical structure (or said another way, the location at which the tubular inner frame 100 is cut to be opened up and flattened for the view shown in FIG. 4).

Another embodiment of an inner frame, in this instance designated 200, is shown in FIG. 5 in a flattened view. Inner frame 200 includes a cylindrical framework 202 defining a lumen 204. As in FIG. 4, this view in FIG. 5 is intended primarily for illustrative purposes of the wireframe structure, and point A and point B designate the locations at which the wireframe is joined to form a cylindrical structure.

Another embodiment of an inner frame, in this instance designated 300, is shown in flattened view in FIG. 6. Inner frame 300 includes three diamond-shaped members, not having spanning members. Cylindrical framework 302 defines a lumen 304 using each of diamond-shaped members 306, 308, and 310. FIG. 6 again shows point A and point B, which are the locations at which the connecting members are joined to form a cylindrical structure.

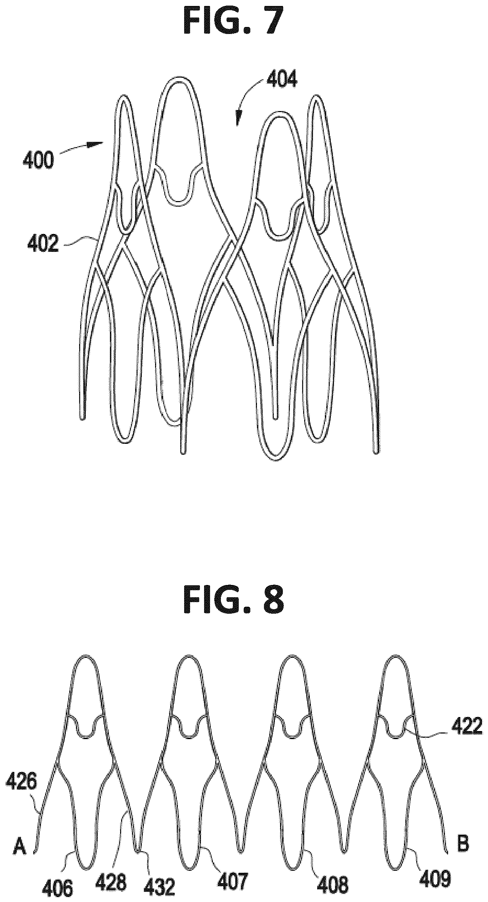

Another embodiment of an inner frame, in this instance designated 400, is shown in side view in FIG. 7. This four-diamond embodiment includes a cylindrical framework 402 defining a lumen 404, in which cylindrical framework 402 includes four generally diamond-shaped members.

A flattened view of another four-diamond embodiment of inner frame 400 is shown in a flattened view in FIG. 8. FIG. 8 shows diamond-shaped members 406, 407, 408, and 409, each having joining legs, shown for 406 as joining components (legs) 426 and 428, which define vertices, such as that shown at joined end 432. FIG. 8 also shows spanning members, such as that shown at 422, crossing the open span of the diamond-shaped members and providing a strengthening structural enhancement, another sewing anchor location, or both. FIG. 8 again shows point A and point B, which are the locations at which the connecting members are joined to form a cylindrical structure.

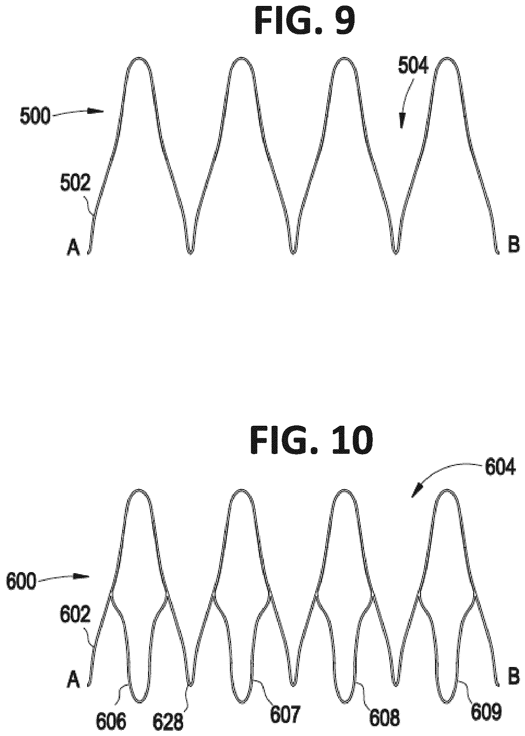

Another embodiment of an inner frame, in this instance designated 500, is shown in flattened view in FIG. 9. Inner frame 500 includes cylindrical framework 502 defining lumen 504.

Another embodiment of an inner frame, in this instance designated 600, is shown in flattened view in FIG. 10. Inner frame 600 includes cylindrical wireframe 602 defining a lumen 604 using diamond-shaped members 606, 607, 608, and 609. Each diamond-shaped member is joined at a connecting point, such as that shown at 628.

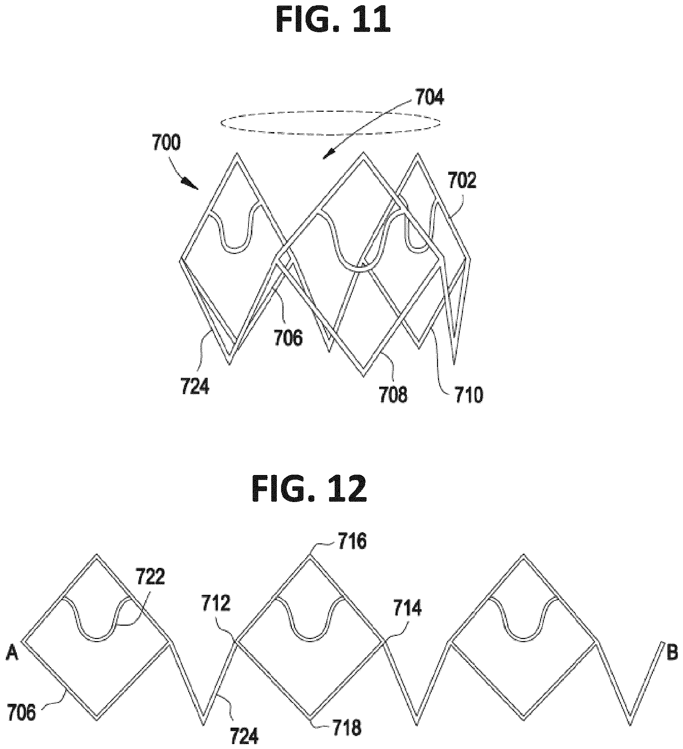

Another embodiment of an inner frame, in this instance designated 700, is shown in side view in FIG. 11 and in flattened view in FIG. 12. This embodiment has three square-shaped members connected by a v-shaped joining element. Square-shaped members 706, 708, and 710, each have a v-shaped joining element, such as that shown as 724. Inner frame 700 also includes lateral vertices 712, 714 and longitudinal vertices 716, 718 and spanning members, such as that shown at 722, crossing the open span of the square-shaped members and providing a strengthening structural enhancement, another sewing anchor location, or both. Again, point A and point B are the locations at which the integral connecting members make their connection to form a cylindrical structure.

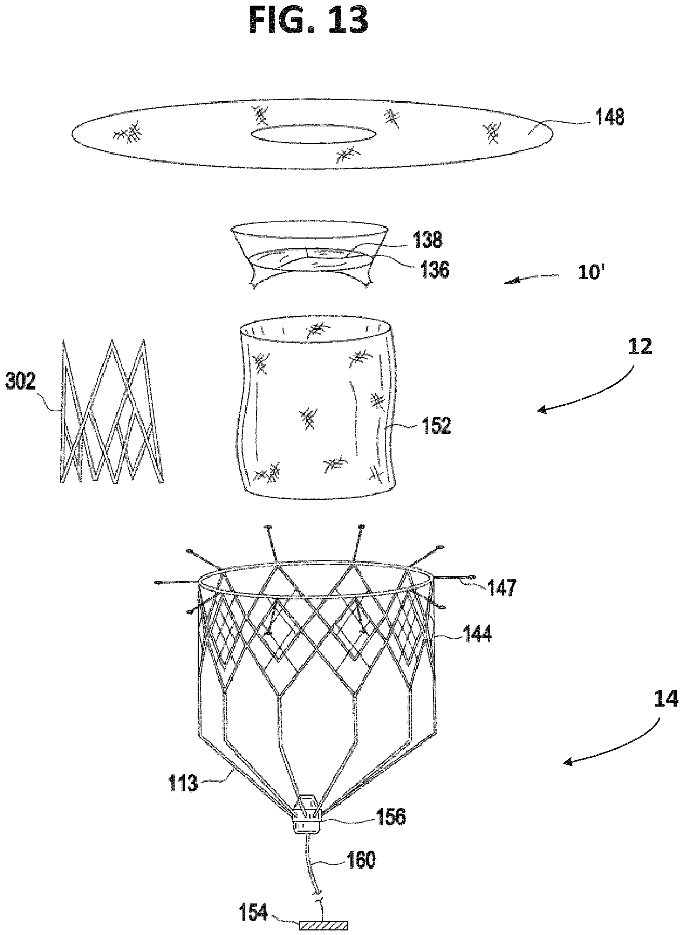

Another embodiment of a prosthetic valve is shown in exploded view in FIG. 13. Valve 10' also includes an inner structure or assembly 12 and an outer structure or assembly 14. Valve 10' may also be coupled to a tether 160 and a tether anchor 154.

Inner assembly 12 includes inner frame 302, outer cylindrical wrap 152, and leaflet structure 136 (including articulating leaflets 138 that define a valve function). As in the embodiment in FIG. 1, leaflet structure 136 may be sewn to inner frame 302, and may use parts of inner frame 302 for this purpose. Inner assembly 12 is disposed within and secured within outer assembly 14.

Outer assembly 14 includes outer frame 144. Outer frame 144 may also have in various embodiments an outer frame cover of tissue or fabric (not pictured), or may be left without an outer cover to provide exposed wireframe to facilitate in-growth. Outer frame 144 has an articulating collar or cuff 147 is covered by cover 148 of tissue or fabric. Cuff 147 may also have in some embodiments a vertical A2 section to accommodate and solve left ventricular outflow tract (LVOT) obstruction issues.

In this embodiment, tether 160 is connected to valve 10' by outer frame 144, in contrast to the embodiment in FIG. 1 , in which the tether is attached to valve 10 by inner frame 100. To implement this alternative tether attachment, in this embodiment, outer frame 144 also has attachment members or struts 113. A tether anchor 156 can be attached to the lower ends of the struts, and tether 160 can be attached to tether anchor 156. In this embodiment, tether 160 can be connected to epicardial securing pad 154.

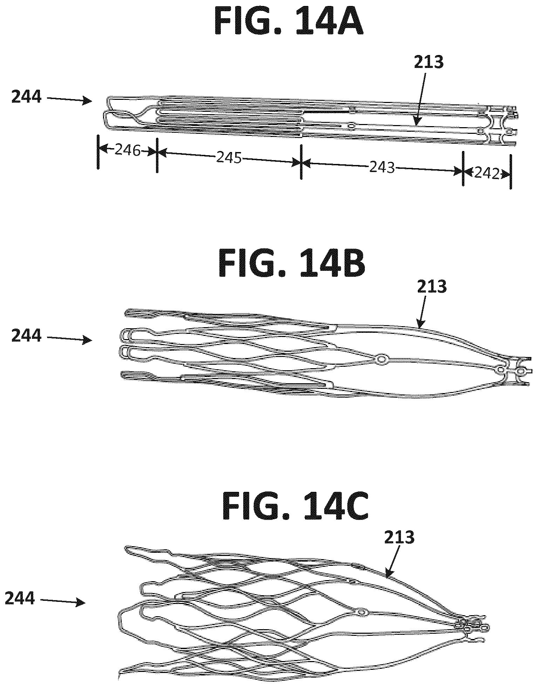

An embodiment of an outer frame 244 having attachment members or struts 213 is shown in FIGs. 14A to 14C. In this embodiment, outer frame 144 is formed from a milled or laser-cut tube of Nitinol^{®}. FIG. 14A shows the tube as initially milled or laser cut, i.e. before deformation. The tube can be divided into four portions, corresponding to functionally different portions of the outer frame 244 in final form: cuff portion 246, frame body portion 245, strut portion 243, and tether connecting portion 242. Strut portion 243 includes six struts 213, which connect body portion 245 to tether clamp portion 242. Connecting portion 242 includes longitudinal extensions of struts 213, connected circumferentially by pairs of opposed, slightly V-shaped connecting members (or "micro-Vs"). Connecting portion 242 is configured to be radially collapsed by application of a compressive force, which causes the micro-Vs to become more deeply V-shaped, with the vertices moving closer together longitudinally and the open ends of the V shapes moving closer together circumferentially. In one embodiment, connecting portion 242 can be configured to compressively clamp or grip one end of a tether, either clamping directly onto a tether line (e.g. braided filament line) or onto an intermediate structure, such as a polymer or metal piece that is in term firmly fixed to the tether line. In another embodiment, connecting portion 242 can be coupled to a tether by a mechanical connection (e.g. stitches, pins, etc.), an adhesive connection, or any other suitable technique. In some embodiments, but clamping and one or more other connection techniques can be used in combination.

In contrast to connecting portion 242, cuff portion 246 and body portion 245 are configured to be expanded radially. Strut portion 243 forms a longitudinal connection, and radial transition, between the expanded body portion and the compressed connecting portion 242. FIG. 14B shows outer frame 244 in a partially deformed configuration, i.e. with connecting portion 242 compressed to a slightly smaller radial dimension than the initial configuration in FIG. 14A, and with cuff portion 246 and body portion 245 expanded to a slightly larger radial dimension. FIG. 14C shows outer frame 244 in a further partially deformed (though not fully deformed to the final, deployed configuration).

Another embodiment of a prosthetic valve is shown in exploded view in FIG. 15. Valve 10" also includes an inner structure or assembly 312 and an outer structure or assembly 314. Valve 10" may also be coupled to a tether 360 and a tether anchor 354.

Inner assembly 312 includes inner frame 340, outer cylindrical wrap 352, and leaflet structure 336 (including articulating leaflets 338 that define a valve function). As in the embodiment in FIGs. 1 and 13, leaflet structure 336 may be sewn to inner frame 340, and may use parts of inner frame 340 for this purpose. Inner assembly 312 is disposed within and secured within outer assembly 314, as described in more detail below.

Outer assembly 314 includes outer frame 370. Outer frame 370 may also have in various embodiments an outer frame cover of tissue or fabric (not pictured), or may be left without an outer cover to provide exposed wireframe to facilitate in-growth. Outer frame 370 may also have an articulating collar or cuff 347 covered by cover 348 of tissue or fabric.

In this embodiment, tether 360 is connected to valve 10" by inner frame 340, similar to the embodiment in FIG. 1, but employing the connecting portion and strut portion features of the outer frame 144 of the valve 10' in FIG. 13. Thus, inner frame 340 includes tether connecting or clamping portion 344 by which inner frame 340, and by extension valve 10", is coupled to tether 360.

Inner frame 340 is shown in more detail in FIGS. 16-18. Inner frame 340 can be formed from a milled or laser-cut tube of, for example, Nitinol^{®}. Inner frame 340 is illustrated in FIG. 16 in an undeformed, initial state, i.e. as milled or laser-cut, but cut and unrolled into a flat sheet for ease of illustration. Inner frame 340 can be divided into four portions, corresponding to functionally different portions of the inner frame 340 in final form: apex portion 341, body portion 342, strut portion 343, and tether clamp portion 344. Strut portion 343 includes six struts, such as strut 343A, which connect body portion 342 to tether clamp portion 344.

Connecting portion 344 includes longitudinal extensions of the struts, connected circumferentially by pairs of micro-V's. Similar to connecting portion 242 of outer frame 244 in FIGs. 14A-C, connecting portion 344 is configured to be radially collapsed by application of a compressive force, which causes the micro-Vs to become more deeply V-shaped, with the vertices moving closer together longitudinally and the open ends of the V shapes moving closer together circumferentially. Thus, connecting portion 344 can clamp or grip one end of a tether, either connecting directly onto a tether line (e.g. braided filament line) or onto an intermediate structure, such as a polymer or metal piece that is in term firmly fixed to the tether line. Other techniques can be used to connect a tether to connection portion 344, as discussed above for connection portion 242 of outer frame 244.

In contrast to connecting portion 344, apex portion 341 and body portion 342 are configured to be expanded radially. Strut portion 343 forms a longitudinal connection, and radial transition, between the expanded body portion and the compressed connecting portion 344.

Body portion 342 includes six longitudinal posts, such as post 342A. The posts can be used to attach leaflet structure 336 to inner frame 340, and/or can be used to attach inner assembly 312 to outer assembly 314, such as by connecting inner frame 340 to outer frame 370. In the illustrated embodiment, the posts include openings through which connecting members (such as suture filaments and/or wires) can be passed to couple the posts to other structures.

Inner frame 340 is shown in a fully deformed, i.e. to the final, deployed configuration, in side view and bottom view in FIGs. 17 and 18, respectively.

Outer frame 370 of valve 10" is shown in more detail in FIGS. 19-21. Outer frame 370 can be formed from a milled or laser-cut tube of, for example, Nitinol^{®}. Outer frame 370 is illustrated in FIG. 19 in an undeformed, initial state, i.e. as milled or laser-cut, but cut and unrolled into a flat sheet for ease of illustration. Outer frame 370 can be similar to outer frame 144 described above in connection with valves 10 and 10'. Outer frame 370 can be divided into a coupling portion 371, a body portion 372, and a cuff portion 373, as shown in FIG. 19.

Coupling portion 371 includes multiple openings or apertures, such as 371A, by which outer frame 370 can be coupled to inner frame 340, as discussed in more detail below.

In this embodiment, cuff portion 373 includes an indicator 374. In this embodiment, indicator 374 is simply a broader portion of the wire frame element of cuff portion 373, i.e. Indicator 374 is more apparent on radiographic or other imaging modalities than the surrounding wire frame elements of cuff portion 373. In other embodiments, indicator 374 can be any distinguishable feature (e.g., protrusion, notch, etc.) and/or indicia (e.g., lines, markings, tic marks, etc.) that enhance the visibility of the part of cuff portion 373 on which it is formed, or to which it is attached. Indicator 374 can facilitate the implantation of the prosthetic valve by providing a reference point or landmark that the operator can use to orient and/or position the valve (or any portion of the valve) with respect to the native valve or other heart structure. For example, during implantation, an operator can identify (e.g., using echocardiography) the indicator 373 when the valve is situated in a patient's heart. The operator can therefore determine the location and/or orientation of the valve and make adjustments accordingly.

Outer frame 370 is shown in a fully deformed, i.e. to the final, deployed configuration, in side view and top view in FIGs. 20 and 21, respectively. As best seen in FIG. 21, the lower end of coupling portion 371 forms a roughly circular opening (identified by "O" in FIG. 21). The diameter of this opening preferably corresponds approximately to the diameter of body portion 342 of inner frame 340, to facilitate coupling of the two components of valve 10".

Outer frame 370 and inner frame 340 are shown coupled together in FIGS. 22-24, in front, side, and top views, respectively. The two frames collectively form a structural support for a prosthetic valve such as valve 10" in FIG. 15. The frames support the valve leaflet structure in the desired relationship to the native valve annulus, support the coverings for the two frames to provide a barrier to blood leakage between the atrium and ventricle, and couple to the tether (by the inner frame 340) to aid in holding the prosthetic valve in place in the native valve annulus by the tether connection to the ventricle wall. The two frames are connected at six coupling points (representative points are identified as "C"). In this embodiment, the coupling points are implemented with a mechanical fastener, such as a short length of wire, passed through aperture (such as aperture 371A) in coupling portion 371 of outer frame 370 and corresponding openings in longitudinal posts (such as poste 342A) in body portion 342 of inner frame 340. Inner frame structure 340 is thus disposed within the outer frame 370 and secured coupled to it.

While various embodiments have been described above, it should be understood that they have been presented by way of example only, and not limitation, and as such, various changes in form and/or detail may be made. Any portion of the apparatus and/or methods described herein may be combined in any suitable combination, unless explicitly expressed otherwise. Where methods and/or schematics described above indicate certain events occurring in certain order, the ordering of certain events and/or flow patterns may be modified. Additionally, certain events may be performed concurrently in parallel processes when possible, as well as performed sequentially.

## Claims

1. An apparatus (10"), comprising:
an outer frame (370) formed of expanded shape memory alloy, the outer frame (370) including a cuff portion (373);
an inner frame (340) coupled to the outer frame (370) and formed of shape memory alloy having a body portion (341, 342) expanded from an initial shape and a connecting portion (344) compressed from an initial shape; and
a tether (360)
the inner frame (340) supporting a prosthetic valve leaflet assembly (336) within the body portion (341, 342),
the connecting portion (344) defining longitudinal extensions of struts connected circumferentially by pairs of opposed V-shaped connecting members, a lumen being defined within the connecting members and configured to engage the tether (360) within the lumen, (1) vertices of the V-shaped connecting members configured to move closer together longitudinally and (2) open ends of the V-shaped connecting members configured to move closer together circumferentially, in response to a compressive force applied to the connecting portion to clamp an end of the tether (360) when the end of the tether (360) is engaged within the lumen,
the end portion of the tether (360) extending from within the lumen of the connecting portion (344) of the inner frame (340) such that an opposite end of the tether (360) can extend and anchor the inner frame (340) to an epicardial surface of a heart when the inner frame (340) is disposed within the heart,
wherein the cuff portion (373) projects beyond a diameter of a tubular body of the outer frame (370).

2. The apparatus (10") of claim 1, wherein the inner frame (340) is connected to the outer frame (370) by mechanical fasteners.

3. The apparatus (10") of claim 1, further comprising a radiopaque indicator (374) formed on the outer frame (370).

## Patentansprüche

1. Vorrichtung (10"), die Folgendes umfasst:
einen äußeren Rahmen (370), der aus expandierter Formgedächtnislegierung ausgebildet ist, wobei der äußere Rahmen (370) einen Manschettenteil (373) einschließt;
einen inneren Rahmen (340), der mit dem äußeren Rahmen (370) verbunden ist und aus Formgedächtnislegierung ausgebildet ist, der einen Körperteil (341, 342), der aus einer Ausgangsform expandiert wird, und einen Verbindungsteil (344), der aus einer Ausgangsform komprimiert wird, aufweist; und
ein Halteband (360),
wobei der innere Rahmen (340) eine Klappensegelprothesenkonstruktion (336) innerhalb des Körperteils (341, 342) stützt,
wobei der Verbindungsteil (344) Längsausdehnungen von Streben definiert, die umlaufend durch Paare von gegenüberliegenden V-förmigen Verbindungelementen verbunden sind, wobei ein Lumen innerhalb der Verbindungselemente definiert ist und konfiguriert ist, um das Halteband (360) innerhalb des Lumens einzulassen, (1) Spitzen der V-förmigen Verbindungelemente konfiguriert sind, um längs näher zusammengeschoben zu werden, und (2) offene Enden der V-förmigen Verbindungelemente konfiguriert sind, um umlaufend näher zusammengeschoben zu werden als Reaktion auf eine Kompressionskraft, die auf den Verbindungsteil ausgeübt wird, um ein Ende des Haltebands (360) festzuklemmen, wenn das Ende des Haltebands (360) innerhalb des Lumens eingelassen wird,
wobei sich der Endteil des Haltebands (360) von innerhalb des Lumens des Verbindungsteils (344) des inneren Rahmens (340) erstreckt, sodass sich ein gegenüberliegendes Ende des Haltebands (360) zu einer epikardialen Oberfläche eines Herzens erstrecken und den inneren Rahmen (340) verankern kann, wenn der innere Rahmen (340) innerhalb des Herzens angeordnet ist,
wobei der Manschettenteil (373) über einen Durchmesser eines rohrförmigen Körpers des äußeren Rahmens (370) hinausragt.

2. Vorrichtung (10") nach Anspruch 1, wobei der innere Rahmen (340) mit dem äußeren Rahmen (370) durch mechanische Befestigungsmittel verbunden ist.

3. Vorrichtung (10") nach Anspruch 1, die weiter einen röntgendichten Indikator (374) umfasst, der an dem äußeren Rahmen (370) ausgebildet ist.

## Revendications

1. Appareil (10"), comprenant:
un cadre externe (370) formé en alliage à mémoire de forme déployé, le cadre externe (370) comprenant une partie de manchette (373) ;
un cadre interne (340) couplé au cadre externe (370) et formé en alliage à mémoire de forme ayant une partie de corps (341, 342) déployée d'une forme initiale et une partie de raccordement (344) comprimée d'une forme initiale ; et
une amarre (360)
le cadre interne (340) soutenant un ensemble de feuillet de valve prothétique (336) dans la partie de corps (341, 342),
la partie de raccordement (344) définissant des extensions longitudinales d'étais raccordées sur la circonférence par des paires de membres de raccordement opposés en forme de V, une lumière étant définie dans les membres de raccordement et configurée pour engager l'amarre (360) dans la lumière, (1) les sommets des membres de raccordement en forme de V étant configurés pour se déplacer plus proches ensemble longitudinalement et (2) les extrémités ouvertes des membres de raccordement en forme de V étant configurées pour se déplacer plus proches ensemble sur la circonférence, en réponse à une force de compression appliquée à la partie de raccordement pour serrer une extrémité de l'amarre (360) lorsque l'extrémité de l'amarre (360) est engagée dans la lumière,
la partie d'extrémité de l'amarre (360) s'étendant de l'intérieur de la lumière de la partie de raccordement (344) du cadre interne (340) de telle sorte qu'une extrémité opposée de l'amarre (360) peut s'étendre et ancrer le cadre interne (340) à une surface épicardique d'un cœur lorsque le cadre interne (340) est disposé dans le cœur,
dans lequel la partie de manchette (373) se projette au-delà d'un diamètre d'un corps tubulaire du cadre externe (370).

2. Appareil (10") selon la revendication 1, dans lequel le cadre interne (340) est raccordé au cadre externe (370) par des attaches mécaniques.

3. Appareil (10") selon la revendication 1, comprenant en outre un indicateur radio-opaque (374) formé sur le cadre externe (370).
